# EUROPEAN PATENT APPLICATION

(11) **EP 4 751 687 A1**
(43) Date of publication of application: **03.06.2026**
(21) Application number: 25218811.5
(22) Date of filing: 26.11.2025
(51) Int. Cl.: A61F 5/02, A61F 5/30

(54) **LUMBAR CORSET**

(30) Priority: 28.11.2024 IT 202400026901; 28.11.2024 IT 202400026910
(71) Applicant: Guasti, Davide, 42049 Sant' Ilario D' Enza (Reggio Nell'Emilia) (IT)
(72) Inventor: GUASTI, Davide, 42049 SANT' ILARIO D' ENZA (REGGIO NELL'EMILIA) (IT)
(74) Representative: Bacchini, Davide

(57) **Abstract**

Lumbar corset (100), comprising:
a main elastic band (1), extending along a main direction (P) between two ends (1a, 1b);
at least one tensioning band (2, 2', 3, 3') extending on the main band (1);
said lumbar corset (100) being configurable in at least one resting condition, in which the two ends (1a, 1b) are distanced from each other and the main band (1) is extended, and an operating condition, in which the two ends (1a, 1b) are at least partially overlapping and the main band (1) takes a substantially annular shape;
in said operating condition, identifying a front part (11) in the main band (1) which is defined by the overlapping of the ends (1a, 1b) and a rear part (12) located on the opposite side.

There are at least four tensioning bands (2, 2', 3, 3') fixed on the main band (1) near or at the rear part (12); said tensioning bands (2, 2', 3, 3') being organised in two pairs, the tensioning bands (2, 2', 3, 3') of each pair having divergent extension therebetween.

## Description

The present invention relates to a lumbar corset. Such a device is also known as a lumbar brace or lumbar band.

In general, such a device for medical use, also called a brace or orthosis, is used for the prevention or treatment of lumbar spine diseases.

Typically, a lumbar corset consists of: a main lumbar band with a front strap closure at the abdominal level; smaller elastic tensioning bands that are fixed with a strap mechanism always in front at the abdominal level to adjust the tension of the main band; reinforcements of the rear (lumbar) and side portions of the main band (splints or pressers of various shapes and materials). These devices aim to reduce the movement of the rachis. In fact, it has been seen that it is not the immobilisation of the rachis that promotes the healing of a large part of diseases, but its rehabilitation in a physiological sense. An example thereof is the well-known "Mckenzie gymnastics", that is, extensional gymnastics that restores and uses lordosis for therapeutic purposes.

Therefore, the objective of these devices should not only be to force the spine to assume a certain position, even the analgesic one assumed for defence, but rather to respect and promote its movement in the physiological sense, that is, in the direction of lordosis (curve with rear concavity and front convexity). In this sense, the function of the corset can also be considered rehabilitative or for functional rehabilitation.

The adjustable elastic tensioning bands used in the known solutions do not follow a preordained direction but are directed from the rear fixing point towards the front, i.e., towards the abdomen, where the adjustable fixing takes place, without particular indications, and always in a single solution, i.e. with only one strap on each side. The user must therefore personally (and without experience) search for the most appropriate tension and position to reduce the movement of the lumbar and benefit therefrom.

Furthermore, as is known, the lumbar corsets currently on the market always have a rear height (lumbar area) greater than the front height (abdominal area) because they reflect the evolution of orthopaedic principles over time.

Once, strong lumbar pain, the cause of which was unknown in the absence of CT and MRI examinations, was immobilised in brace casts that wrapped around the trunk as a whole (chest/back and abdomen/loins). These rigid braces respect the curves of the spine, dorsal kyphosis, lumbar lordosis.

The modern version of these rigid braces is represented by metal orthoses or rigid materials that aim to immobilise the rachis while maintaining or overcorrecting (hyper-extension) the curvature. The most common function of these braces is the treatment of vertebral fractures. They have a size such that the point of support and thrust always goes beyond the lumbar alone.

Modern non-rigid lumbar corsets represent the reduced shape of the aforementioned rigid braces to the lumbar alone.

In this context, the Applicant has found numerous cases of patients with lumbar pain who do not benefit from the non-rigid lumbar brace. After a series of studies, he has identified that the main problem of non-rigid lumbar corsets lies in the fact that, although they are limited to the lumbar area alone, they maintain the height relationships (rear height near the rachis greater than front height) of the rigid braces, consequently losing the original respect of the curves.

This drift is certainly also favoured by the sedentary lifestyle that forces people to a sitting position for a large part of the day, whereby support for high back pain in the front (on the belly) would create discomfort and impediment.

The lumbar curve is a curve with rear concavity and front convexity. A support which is higher in the rear than in the front translates, from the biomechanical point of view, into a condition of opposition to lordosis, that is, it goes against rachis well-being.

In fact, a non-rigid lumbar brace of the prior art tends to immobilise the loins in kyphosis or in general to counteract lordosis, which is the biomechanical condition that the back needs.

In this context, the technical task underlying the present invention is to propose a lumbar corset, which allows to overcome the limits of the prior art.

In particular, an object of the present invention is to provide a lumbar corset which promotes lumbar lordosis.

Another object of the present invention is to propose a lumbar corset suitable for gymnastics and back rehabilitation.

The stated technical task and specified objects are substantially achieved by a lumbar corset comprising the technical features disclosed in one or more of the appended claims.

Further features and advantages of the present invention will become more apparent from the illustrative and thus non-limiting description of a preferred but not exclusive embodiment of a lumbar corset as illustrated in the appended drawings, in which:

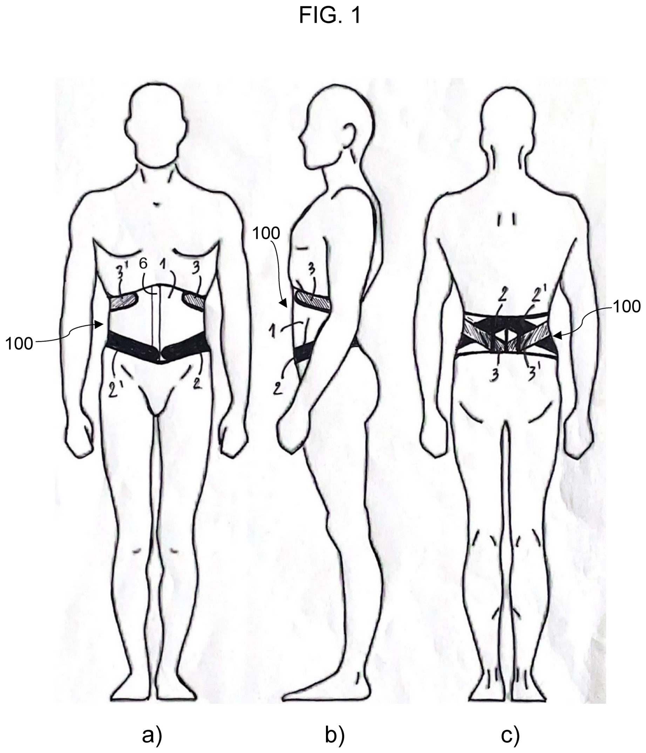

- Figures 1a, 1b and 1c illustrate a lumbar corset, according to the present invention, applied to a user (operating condition), respectively in a front, side and rear view;

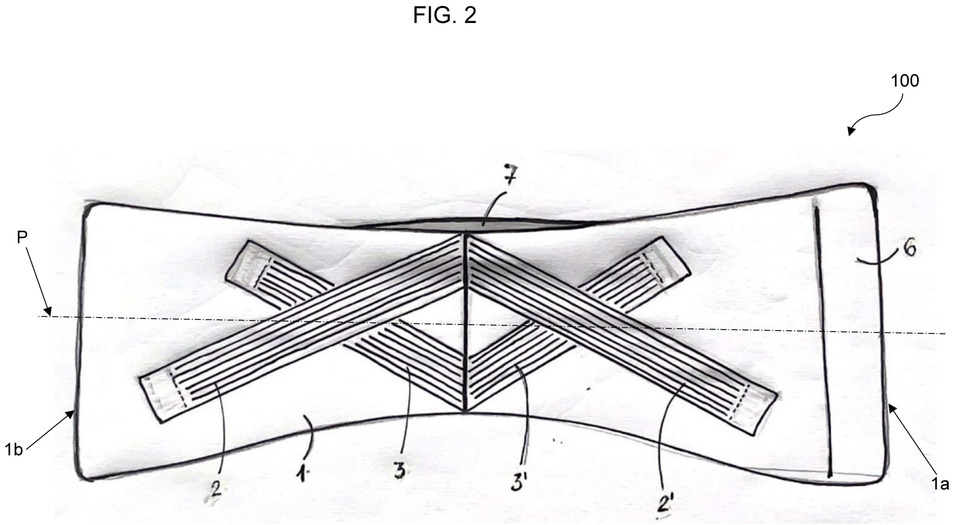

- Figure 2 illustrates the lumbar corset of Figure 1 in resting condition, in front view;
- Figures 3a, 3b and 3c illustrate an embodiment (functional band) of the lumbar corset, applied to a user (operating condition), respectively in front, side and rear view;
- Figure 4 illustrates the lumbar corset of Figures 3a, 3b and 3c, in perspective view from behind;
- Figures 5a and 5b illustrate a phase of use of the functional band of the lumbar corset of Figure 3a, 3b and 3c, respectively in side and rear view;
- Figure 6 illustrates a human figure in side view with the spine visible, in which the lumbar can be distinguished and the natural divergence of the lines that delimit this curve and at the same time the approximate shape of the lumbar corset that is determined are amplified.

With reference to the figures, the reference numeral 100 indicates a lumbar corset according to the present invention.

The lumbar corset 100 comprises a main elastic band 1 and at least one elastic tensioning band 2, 2', 3, 3' extending on the main band 1. In particular, the tensioning band is applied on a face of the main band 1 and extends along it.

In particular, the main band 1 constitutes the basic scaffolding of the lumbar corset 100, while the tensioning band 2, 2', 3, 3' serves for adjustment.

The main band 1 extends along a main direction "P" between two ends 1a, 1b. Suitably, the main direction "P" corresponds in use to the wrapping direction of the lumbar corset 100 around the user's body.

The lumbar corset 100 is configurable in at least one resting condition, in which the main band 1 is extended and the two ends 1a, 1b are distanced from each other, and an operating condition, in which the two ends 1a, 1b of the main band 1 are at least partially overlapping and the main band 1 takes a substantially annular shape.

That is, conventionally in the resting condition the corset is defined as open, while in the operating condition as closed.

In the operating condition, a front part 11 is thus formed which is defined by the overlapping of the ends 1a, 1b and a rear part 12 located on the opposite side of the main band 1 with respect to the front part 11.

In particular, in the resting condition the rear part 12 corresponds to a central area of the main band 1.

It should be noted that, for the purposes of this invention, the term "central area" refers to a portion of the main band 1 that is not close to the ends 1a, 1b and is large enough to substantially cover the user's back.

For clarity, a "middle are" of the main band 1 will also be identified. This expression, which will be used in the description, refers to a narrower portion of the so-called "central area" and is located around the medial part of the length of the main band 1 when stretched out. In use, the middle area substantially corresponds to the central part of the back where the spine is located.

By convention, the abdominal part is defined as the "front part" of the corset, i.e., lying on the user's abdomen, while the "rear part" is the lumbar part, i.e., lying on the loins. This conventional definition of front and rear is generally valid for all corsets on the market, therefore well known to a person skilled in the art.

The lumbar corset 100 comprises four tensioning bands 2, 2', 3, 3' or so-called pull straps. In particular, each tensioning band 2, 2', 3, 3' extends between a fixed end and a free end.

The tensioning bands 2, 2', 3, 3' are fixed to the rear of the main band 1. In other words, the fixed end of the tensioning bands is located at the rear part 12 of the corset 100.

"Rear" means at or near the central area in the resting condition, which corresponds to the rear part 12 in the operating condition. In other words, "rear" means the entire area covering the user's back.

The four tensioning bands 2, 2', 3, 3' are organised in two pairs. The two tensioning bands 2, 2', 3, 3' of each pair have divergent extension therebetween.

As mentioned, the tensioning bands are adjustable, that is, pulled frontwards by the user to achieve the desired tension.

Preferably, the free ends of the tensioning bands 2, 2', 3, 3' are removably anchorable in front of the main band 1, for example by means of a strap fixing system.

In other words, the tensioning bands 2, 2', 3, 3' are independent in the end anchorage point.

In particular, the tensioning bands 2, 2', 3, 3' are anchorable laterally or at the front.

"In front" means near or at the ends 1a, 1b in the resting condition, which corresponds to the front part 11 in the operating condition.

"Laterally" means an intermediate position between the front part 11 and the rear part, which corresponds to the user's flank.

In other words, the two bands of each pair are free and independent in their course, as well as divergent, and may end at different points on the lateral or front side.

Preferably, the tensioning bands 2, 2', 3, 3' originate from the central area 12.

In accordance with an embodiment, the tensioning bands 2, 2' of one pair have divergent extension in the opposite direction to that of the tensioning bands 3, 3' of the other pair.

In more detail, the opposite direction refers to a component of substantially orthogonal extension in the main direction "P", that is, a component along the height.

Preferably, the tensioning bands 2, 2' of one pair have divergent extension downwards, while the tensioning bands 3, 3' of one pair have divergent extension upwards.

"Downwards" means an extension along the main band 1 having a component along the height which decreases. "Upwards" instead means an extension along the main band having a component along the height which increases.

In other words, the four tensioning bands 2, 2', 3, 3' are divergent in the posterior-anterior direction, two on each side. Side means the part of the main band 1 that connects the central area 12 to one of the ends 1a, 1b. The aforesaid organisation of the four tensioning bands provides an effective lumbar-supporting effect.

In the rear point, this effect can be achieved even if the bands are overlapped in whole or in part, if the height of the main band is low. However, the embodiment described below provides better efficacy. Preferably, the tensioning bands 2, 2' of one pair originate in the middle area from a region which is located higher than that from which the tensioning bands 3, 3' of the other pair originate.

Since they have a divergent extension towards the front part 11, i.e., towards the ends 1a, 1b, the fact that a pair of tensioning bands 3, 3' originates from a lower region means that its tensioning bands 3, 3' are located at the front part 11 in a higher position than the tensioning bands 2, 2' of the other pair.

Two upper bands 3, 3' and two lower bands 2, 2' are therefore identified in relation to the position they assume at the front part 11, i.e., in use with respect to the front view of the user.

Therefore, the two upper bands 3, 3' have divergent extension upwards, while the two lower bands 2, 2' have divergent extension downwards. Preferably, the two upper bands 3, 3' originate from a lower end point of the middle area, while the two lower bands 2, 2' originate from an upper end point of the middle area.

The fact that the rear starting point of the pairs of bands is inverted (upper for the lower bands 2, 2' and lower for the upper bands 3, 3') allows the degree of divergence thereof to be increased so as to give the user the clear sensation of a forward thrust of the abdomen, a sign of the increase in the lumbar curve.

Preferably, the two upper bands 3, 3' are shorter than the lower ones 2, 2'. This is because the upper bands do not have to reach the median line, they are fixed at the side edge of the rectus abdominis (para-rectal line). The two lower bands 2, 2' are longer because they reach the median line and can also exceed it. They are fixed at the pubic region.

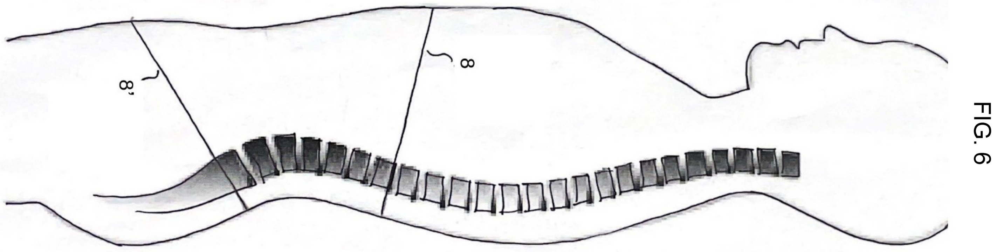

In general, the height of the main band 1 must be sufficient to allow the course of the bands to be effectively divergent, i.e., to respect the natural divergence 8, 8' indicated by the vertebral bodies that describe the lumbar curve, as can be clearly seen from Figure 6.

In accordance with an embodiment, the tensioning bands 2, 2', 3, 3' originate at an anchoring line located in the medial part of the main band 1, that is at the middle are.

In accordance with an embodiment, the tensioning bands 2, 2', 3, 3' originate at an anchoring line which also corresponds to the section of the main band 1 having a smaller height.

Preferably, the lumbar corset 1 above described thus have two tensioning bands 2, 2', 3, 3' per side which are independent in their end anchorage point, that is of the free ends.

In accordance with an embodiment, the main band 1 is shaped so that, in the operating condition, the front part 11 has a greater size along a direction substantially orthogonal to the main direction "P" than the rear part 12.

Therefore, the size of the main band 1 along the main direction "P" is defined as the "length" of the lumbar brace 100 and the size in a direction orthogonal to the latter is defined as the "height". Consequently, the main band 1 in operating condition has a height in the front part 11 greater than the height in the rear part 12.

As is known, a band is a predominantly two-dimensional element, i.e., the third dimension (conventionally "thickness") is negligible compared to the other two mentioned above.

Thereby, a lumbar corset is obtained in which the front height is greater than the rear one in the main band, thus favouring lumbar lordosis.

In use, the direction substantially orthogonal to the main direction "P", called height, corresponds to a vertical direction, i.e., following the development of the user's spine.

Preferably, the main band 1 is shaped so that in the resting condition the ends 1a, 1b each have a greater size along a direction substantially orthogonal to the main direction "P" (i.e., along the height) than the central area of the main band 1. Reference is made to the above comments regarding the term "central area", specifying that it refers to a position along the main direction "P" relative to ends 1a and 1b.

It is therefore noted that the central area in the resting condition, i.e., when the main band 1 is extended, corresponds to the rear part 12 in the operating condition, i.e., when the main band 1 is closed in a loop.

Therefore, it will be indicated with the same numerical reference 12.

In accordance with an embodiment, the main band 1 extends between the middle area and the end 1a, 1b with a progressive increase in size along a direction substantially orthogonal to the main direction "P" (i.e., along the height).

Preferably, this applies to both ends 1a, 1b. That is, the middle area has the smallest size along a direction substantially orthogonal to the main direction "P" (i.e., along the height). Starting from the middle area, the main band 1 extends towards both ends 1a, 1b with a progressive increase in height.

In general and by way of example, the rear height typically does not exceed 14 centimetres. However, the numerical data depends on the anthropometric values of the user.

In accordance with the present invention, it must necessarily be lower than the front height so as to allow a divergent course of the bands and the perception of a difference by the user. The lower the height, the greater the chance of developing the desired lumbar-lowering effect. On the contrary, the greater the height, the greater the risk of an unfavourable action.

Therefore, corsets with a rear height even greater than 14 centimetres may exist, as long as the front height is greater.

The anatomical and physiological reference for these quantitative evaluations is represented by the motion segment, which is the elementary unit of movement of the rachis and the overall extension of the lumbar. The motion segment consists of two contiguous vertebrae plus the interposed intervertebral disc. Operationally, it approximates to about 8 centimetres, due to the enormous variability of humans.

The lordosis (evident in Figure 6) is defined and must be respected for each individual motion segment, but above all for the lumbar rachis as a whole (five vertebrae to which are added, from the functional point of view, the adjacent vertebrae, i.e., D12 and S1). The overall extension of the indicated lumbar is around 28 centimetres. Therefore, the height of 10-14 centimetres, in any case not exceeding 14 centimetres, is to be considered a reasonable average value able to respect the spine as a whole. The value is much lower than the overall height of the lumbar rachis and at the same time is only slightly higher than the height of a motion segment. In fact, it should be considered that the height on the skin, from the mechanical point of view, i.e., the pressure exerted, does not correspond to the same height on the rachis, since this is attenuated by the distance that exists between the two structures. For this reason, a height of 10-14 centimetres on the skin can correspond to a height of about 8 centimetres on the rachis.

It should be noted that the lumbar corset acts on the lumbar region, which is scientifically defined as the anatomical section corresponding to the five lumbar vertebrae and the surrounding muscles, in particular those of the loins. The lumbar region does not therefore end at the midline where the vertebrae are located, but extends laterally to a lateral groove that corresponds to the boundary of the muscles, and is bounded above by the twelfth rib and below by the iliac crest. The height of a device that is applied to the lumbar region, such as a corset, cannot therefore be referred to only to the midline but to the entire region.

Furthermore, the lumbar region is not a neutral, flat anatomical structure, but rather a curve. The height of the lumbar corset in operational condition is therefore counter-shaped to the lumbar curve as a whole. It is therefore a biomechanical height, as the lumbar corset 1 of the present invention is designed to act on this curve, promoting its function. For this reason, it is lower at the back than at the front in order to act in accordance with the biomechanics of the curve, which is healthy when its lordosis is respected. The biomechanical height is therefore not an average of the heights that can be measured on the lumbar region but is the highest height that can be found on it. If there were a lower height on the midline than on the front, but immediately to the side of it this increased to a higher value than on the front, then the corset would not produce the effects described in this invention.

Therefore, when describing in this invention that the height of the rear part 12 is lower than the height of the front part 11, it is understood that every point on the rear surface or lumbar region, or better still, the rear view of the trunk, must necessarily be lower than the maximum height of the front surface or abdominal region or front view. In other words, the rear part 12 has heights that are lower than the maximum height of the front part 11. The rear part 12 of the lumbar corset 1 is to be understood as the entire portion of the main band 1 that is intended to contact the back of a user. In other words, the rear part 12 is a portion of the main band 1, opposite the front part 11, which continuously presents a plurality of adjacent sections, all having a height lower than the maximum height of the front part 11. Preferably, in the rear part in a central position, a pocket 7 is positioned for housing a reinforcement made of rubber or other rigid or semi-rigid material as well as for devices adapted to produce heat or generate electromagnetic fields for the treatment of low back pain.

The presence of a rear reinforcement (i.e., at the central area) inserted in a special pocket with a larger axis in the line of the main axis of the corset allows to reduce the area of actual thrust, concentrating it on a smaller surface both on the user's skin and on the rachis.

The front height must in any case be greater than the rear one. The higher the front part, the greater the lumbar-supporting effect that is obtained. In any case, it will not extend beyond the abdominal surface, as is the case with rigid braces, i.e., the chest.

The lumbar corset 1 with diverging tension bands 2, 2', 3, 3' and a main band 1 with different front and rear heights is particularly advantageous for a synergistic effect of activating different mechanisms:
- proprioceptive stimulation (cutaneous and musculoskeletal stimulation received by the CNS) linked to the shape of the main band which is precisely higher in front;
- the thrust of smaller adjustable elastic bands, diverging in a posterior-anterior direction.

However, from the above description of the embodiment of the main band 1 with different front and rear heights, it is immediately clear that this embodiment refers to a construction principle of the corset which, in itself, is independent of the tensioning bands and produces its own technical effects (lordosis, band divergence, ergonomics). Therefore, it is not exclusively linked to the characteristic of diverging tensioning bands, nor is it inseparable from them in terms of function and structure.

Suitably, the main band 1 comprises a strap closure system 6 for the ends 1a, 1b. A system of this type is widely known in the field, therefore it will not be further detailed.

In the embodiment illustrated in the figures, the ends 1a, 1b comprise at least one Velcro^{®} strip.

In this case, the strap closure of the two ends takes place at the front part 11, while the rear part 12 has no continuity solutions (corresponds to the central part of the main band 1).

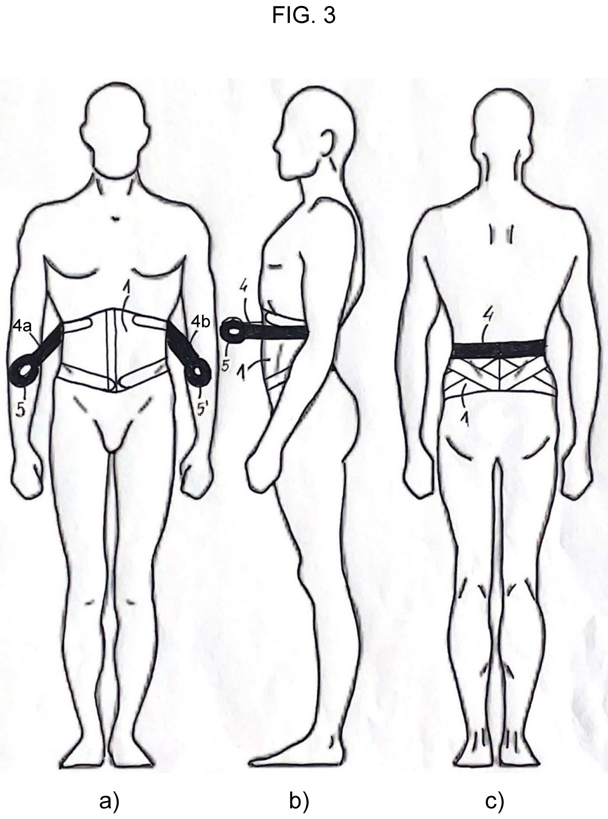

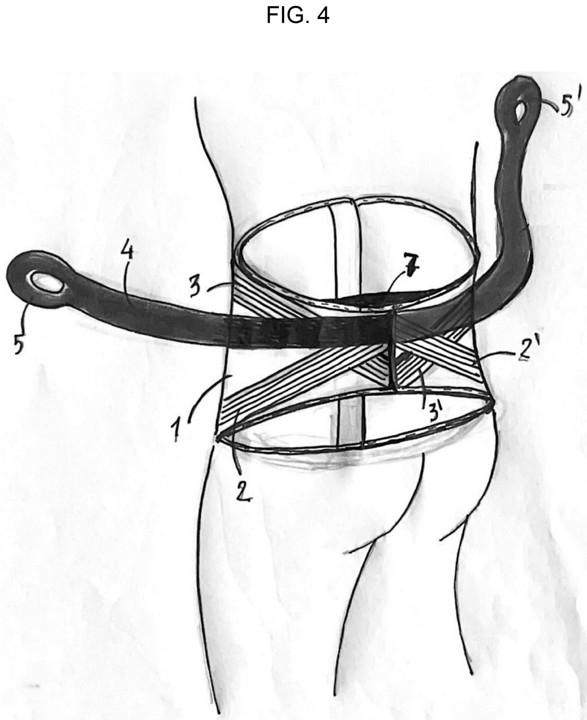

In accordance with an embodiment, illustrated in Figures 3-5, the lumbar corset 100 comprises a functional elastic band 4 anchored to the main band 1 and defining two freely projecting arms 4a, 4b.

With reference to the anchoring point, one arm 4a is the right part of the functional band 4 and the other arm 4b is the left part.

Each arm 4a, 4b ends with a gripping element 5 for the user's hands. Preferably, the gripping element 5 is a loop.

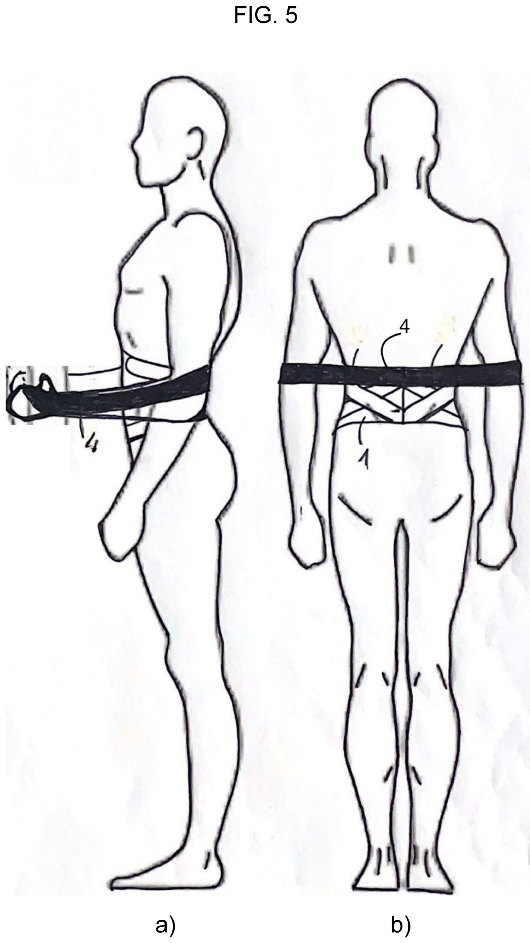

In particular, the functional band 4 is rigid or semi-rigid, fixed or removable. The course of the band, during use, entails that it surrounds the elbow and forearm, on both sides, before reaching the hands (as illustrated in Figure 5).

The function of this band is rehabilitative and functional. It can serve to further increase, thanks to the action of the arms, the lumbar curve (McKenzie-type exercises in a seated position) during the fixed upright position or allow an alternating movement of the arms and legs during walking with associated torsion of the trunk.

The present invention has numerous advantages.

The lumbar corset which is the subject matter of the invention can be defined as functional since it respects the physiology of the lumbar with particular reference to respect for lordosis. Furthermore, the proposed corset can be used not only to reduce mobility, the main objective of each lumbar corset, but also to promote rehabilitation and back gymnastics. This is not possible with the existing lumbar corsets.

The lumbar corset of the invention, in which the front height is greater than the rear one in the main band, favours lumbar lordosis. In particular, each additional element puts a different mechanism into action:
- proprioceptive stimulation (cutaneous and musculoskeletal stimulation received by the CNS) linked to the shape of the main band which is precisely higher in front;
- the thrust of smaller adjustable elastic bands, diverging in a posterior-anterior direction and fixed to the corset both in the front and rear;
- the thrust of an additional band (said functional band), fixed to the corset only in the rear for the front hand grip;
- the presence of a rubber reinforcement inserted in a rear pocket.

## Claims

1. A lumbar corset (100), comprising:
a main elastic band (1), extending along a main direction (P) between two ends (1a, 1b);
at least one tensioning band (2, 2', 3, 3') extending on the main band (1); said lumbar corset (100) being configurable in at least one resting condition, in which the two ends (1a, 1b) are distanced from each other and the main band (1) is extended, and an operating condition, in which the two ends (1a, 1b) are at least partially overlapping and the main band (1) takes a substantially annular shape;
in said operating condition, identifying a front part (11) in the main band (1) which is defined by the overlapping of the ends (1a, 1b) and a rear part (12) located on the opposite side,
wherein there are at least four tensioning bands (2, 2', 3, 3') fixed on the main band (1) near or at the rear part (12); said tensioning bands (2, 2', 3, 3') being organised in two pairs, the tensioning bands (2, 2', 3, 3') of each pair having divergent extension therebetween.

2. The lumbar corset (100) according to claim 1, wherein the tensioning bands (2, 2', 3, 3') of one pair have divergent extension in the opposite direction to that of the tensioning bands (2, 2', 3, 3') of the other pair.

3. The lumbar corset (100) according to claim 1 or 2, wherein the tensioning bands (2, 2', 3, 3') of one pair have divergent extension downwards and the tensioning bands (2, 2', 3, 3') of the other pair have divergent extension upwards.

4. The lumbar corset (100) according to any one of the preceding claims, wherein each pair originates at a middle area of the main band (1).

5. The lumbar corset (100) according to claim 4, wherein the tensioning bands (2, 2') of one pair originate in the middle area from a region which is located higher than that from which the tensioning bands (3, 3') of the other pair originate.

6. The lumbar corset (100) according to any one of the preceding claims, wherein the main band (1) is shaped such that, in the operating condition, the front part (11) has a greater size along a direction substantially orthogonal to the main direction (P) than the rear part (12).

7. The lumbar corset (100) according to claim 6, wherein the main band (1) is shaped so that, in the resting condition, the ends (1a, 1b) each have a greater size along a direction substantially orthogonal to the main direction (P) than a central area of the main band (1), said central area corresponding in the operating condition to the rear part (12).

8. The lumbar corset (100) according to claim 6 or 7, wherein the main band (1) extends between the central area (12) and the end (1a, 1b) with a progressive increase in size along a direction substantially orthogonal to the main direction (P).

9. The lumbar corset (100) according to any one of the preceding claims, wherein the tensioning bands (2, 2', 3, 3') are independent in their end anchoring points.

10. The lumbar corset (100) according to any one of the preceding claims, wherein the main band (1) comprises a pocket (7) in the rear part (12) for housing a reinforcement or devices adapted to produce heat or generate electromagnetic fields.
